# EUROPÄISCHE PATENTSCHRIFT

(11) **EP 1 067 902 B1**
(45) Veröffentlichungstag und Bekanntmachung des Hinweises auf die Patenterteilung: **16.04.2003**
(21) Anmeldenummer: 99917911.2
(22) Anmeldetag: 31.03.1999
(51) Int. Cl.: A61K 7/48, A61K 7/027, A61K 7/032

(54) **KOSMETISCHE FORMULIERUNG MIT VERÄNDERBAREM VOLUMEN**
COSMETIC FORMULATION WITH A VARIABLE VOLUME
FORMULATION COSMETIQUE A VOLUME VARIABLE

(30) Priorität: 09.04.1998 DE 19817522
(43) Veröffentlichungstag der Anmeldung: 17.01.2001
(73) Patentinhaber: Coty B.V., 2031 CC Haarlem (NL)
(72) Erfinder: MOHR, Celia, Somerset, NJ 08873 (US); MACCHIO, Ralph, Sparta, NJ 07971 (US); BARONE, Salvatore, Staten Island, NY 10314 (US); GUERRERO, Alberto, Orangeburg, NY 10962 (US)
(74) Vertreter: Walter, Wolf-Jürgen
(86) Internationale Anmeldenummer: EP9902161
(87) Internationale Veröffentlichungsnummer: WO99052499

(56) Entgegenhaltungen:
- EP-A- 0 195 575
- US-A- 3 574 822
- US-A- 3 697 643
- US-A- 5 091 013
- KLEIN W L ET AL: "ACRYLATES COPOLYMER: A TECHNIQUE FOR ENTRAPPING COSMETIC ACTIVES" HAPPI HOUSEHOLD AND PERSONAL PRODUCTS INDUSTRY, Bd. 26, Nr. 7, 1. Juli 1989 (1989-07-01), Seite 118, 120, 122, 124 XP000068293 ISSN: 0090-8878
- PATENT ABSTRACTS OF JAPAN vol. 009, no. 218 (C-301), 5. September 1985 (1985-09-05) & JP 60 081120 A (SHISEIDO KK), 9. Mai 1985 (1985-05-09)

## Beschreibung

Die Erfindung betrifft eine kosmetische Formulierung mit veränderbarem Volumen.

Für verschiedene kosmetische Anwendungszwecke z.B. bei Schminken ist es erwünscht, bestimmte Hautpartien insbesondere im Gesicht voller erscheinen zu lassen. Üblicherweise wird das durch spezielle dick erscheinende Massen, besondere Make-up's, mehrfaches Auftragen, halbchirugische oder chirugische Maßnahmen, Einspritzen bestimmter Flüssigkeiten usw. erreicht. Alle diese Maßnahmen weisen jedoch Nachteile auf, da sie entweder problematisch für die Haut des Anwenders sein können, oder ihre Struktur nicht die gewünschte Formbeständigkeit über längere Zeiträume zeigt.

Aus der EP-A-195575 ist eine cremige Lippenstiftformulierung bekannt, die als Wirkstoff Acrylates Copolymer enthält und eine verbesserte Auftragbarkeit aufweist. In der US-A-5091013 wird die Erhöhung des Feuchthaltevermögens von Pigmentteilchen durch eine Ummantelung mit einer hochmolekularen Verbindung mit -COOR-Gruppen (R=H, Al, Mg, Ca, Zn, Zr, Ti) erreicht. Aus Patents Abstracts of Japan vol. 009, No. 218 (C-301) 1985-09-05 & JP-A-60 081 120) sind Hochpolymere wie selbstvernetzendes Natriumpolyacrylat zur Absorption von Wasser in kosmetischen Zubereitungen bekannt.
Der Erfindung liegt die Aufgabe zugrunde, eine dauerhafte und für die Haut, insbesondere für die Gesichtshaut annehmbare und wirksame kosmetische Formulierung mit Volumenvergrößerung zu entwikkeln.

Eine spezielle Aufgabe der Erfindung liegt in der Bereitstellung eines Lippenstiftes mit Volumenvergrößerung.

Erfindungsgemäß ist die kosmetische Formulierung mit Volumeneffekt gekennzeichnet durch einen Gehalt an
a) einem Poly(natriumacrylat)-Homopolymeren, einem Natriumsalz von Polyacrylsäure oder einem Gemisch davon, das bei Kontakt mit Feuchtigkeit oder Wasser sein Volumen bis zum 5-fachen seines ursprünglichen Volumens vergrößert, im Bereich von 0,01 bis 90 Gew-%;
b) einem Erweichungsmittel, umfassend kosmetisch annehmbare Öle, Ester, Silikonflüssigkeiten und deren Gemische im Bereich von 0,1 bis 95 Gew-%;
c) Wachs, umfassend Carnaubawachs, Candelillawachs, Ozokerit, Ceresin, Paraffin, synthetischen Wachs und Gemische davon im Bereich von 0,1 bis 50 Gew-%;
d) Öl, umfassend Rizinusöl, Lanolinöl, synthetisches Öl, Silikonöl und Gemische davon im Bereich von 0,5 bis 90 Gew-%;
e) weitere Wirk- und Hilfsstoffe, wie Antioxidationsmittel, Fette, Fettsäureester, Lanolin, Lanolinderivate, Farben, Pigmente, Parfüm, Schutzmittel, Glimmer, Talkum, Siliciumdioxid, synthetische Harze, Polymere, die einen restlichen Anteil bis zu 100 Gew-% ausmachen;
wobei die Prozentangaben auf die Gesamtformulierung bezogen sind und wobei die Formulierung einen Wassergehalt von kleiner als 0,01 Gew-% hat.

Die Teilchengröße des Poly(natriumacrylat)-Homopolymeren, des Natriumsalzes der Polyacrylsäre oder des Gemisches beider liegt vorteilhaft unter 200 µm.

Ein besonders bevorzugter Komplex für den Einsatz in der erfindungsgemäßen Formulierung ist ein separat hergestelltes Gemisch aus Polyacrylat-Polymerem, z.B. Natriumpolyacrylat, einem Öl wie Rizinusöl und einem festen Antioxidationsmittel wie Tocopherylacetat. Dabei liegt der Anteil des Antioxidationsmittels zwischen 0,5 und 2 Gew-%, und der Anteil der anderen beiden Komponenten ist etwa gleich.

In einer Ausführungsform der Erfindung kann der Anteil des Polyacrylat-Polymeren im Bereich von 0,1 bis 60 Gew-% liegen, in anderen Ausführungsformen im Bereich von 0,1 bis 40 Gew-% oder 0,1 bis 20 Gew-%, jeweils bezogen auf die Gesamtmasse der kosmetischen Formulierung.

Eine besonders bevorzugte Ausführungsform der Erfindung enthält ein Polyacrylat-Polymeres in einer Menge, die eine Volumenausdehnung der Gesamtformulierung bei Kontakt mit Feuchtigkeit oder Wasser auf bis zu 150 % des ursprünglichen Volumens ermöglicht.

Die in der Formulierung als Erweichungsmittel eingesetzten kosmetisch annehmbaren Öle, Ester und Silikonflüssigkeiten umfassen vorzugsweise Isoeicosan, Dimethicone, Myristylmyristat und Behenylerucat, wobei auch andere Materialien, wie z.B. pflanzliche Öle, eingesetzt werden können, sofern sie innerhalb des Gesamtsystems zu keinen nachteiligen Wirkungen führen. Der Gehalt an Erweichungsmitteln liegt vorteilhaft im Bereich von 1 bis 60 Gew-%, vorzugsweise 2 bis 50 Gew-%.

Wie bereits ausgeführt, kann die Formulierung weitere Wirk- und Hilfsstoffe enthalten, zu denen auch Alkohole, Polyole, Stearinsäure, Magnesiumstearat, Cetearyloctanoat, Maisstärke und organische Lichtschutzmittel gehören können. Anstelle der oben genannten Wachse oder im Gemisch mit diesen können auch Wachse wie Bienenwachs und Montanwachs verwendet werden.

Zu einsetzbaren öllöslichen UVB-Filtern als Lichtschutzmittel gehören z.B. 4-Aminobenzoesäure-Derivate wie der 4-(Dimethylamino)-benzoesäure-(2-ethyl-hexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Pigmente, Pigmentgemische oder Pulver mit pigmentartiger Wirkung, worunter auch solche mit Perlglanz-Effekt zu verstehen sind, können zum Beispiel umfassen Eisenoxide, Titanoxid (TiO₂), Glimmer, Kaolin, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, verkapselte und unverkapselte Getreidestärken sowie Glimmer-Titanoxid-organischer Farbstoff, ZrO₂, MnO, Al₂O₃ usw., die auch im Gemisch eingesetzt werden können.

Die erfindungsgemäße Formulierung liegt in Form eines Lippenstiftes, einer Grundierung, einer Wimperntusche, eines Make-up's, eines Kaschierstiftes und ähnlichem vor.

Von den erfindungsgemäß verwendeten Polyacrylaten ist bekannt, daß sie u.a. in Sanitärprodukten, wie Windeln und Monatsbinden usw. als saugfähiges Material eingesetzt werden können. Die Verwendung in der Kosmetik ist neu und erforderte die Lösung einer Reihe von Problemen, wie die Einstellung des Systems ohne Ausfällungen oder Agglomerierungen und das Arbeiten ohne jegliche Anwesenheit von Wasser. Überraschenderweise wurde gefunden, daß mit einem Gemisch von Erweichungsmitteln,
Wachsen und ölen sowie ausgewählten weiteren Wirk- und Hilfsstoffen eine stabile kosmetische Formulierung erhalten werden kann, die sowohl bei höherem Wachsanteil zu Stiften wie Lippenstiften, als auch bei sehr niedrigem Wachsanteil zu pastenartigen Formulierungen verarbeitet werden kann.

Eine spezielle Ausführungsform der Erfindung betrifft einen Lippenstift mit einem Wachsanteil von 8 bis 15 Gew-%, einem Ölanteil von 22 bis 32 Gew-%, einem Anteil an Polyacrylat-Polymerem von 4 bis 12 Gew-%, einem Anteil an Erweichungsmittel von 2 bis 20 Gew-% und dem restlichen Anteil weiterer Wirk- und Hilfsstoffe.

Ein weiterer Gegenstand der Erfindung ist ein Verfahren zur Herstellung einer kosmetischen Formulierung mit Volumeneffekt, dadurch gekennzeichnet, daß man ein homogenes Gemisch aus Poly(natriumacrylat)-Homopolymerer, ein Natriumsalz von Polyacrylsäure oder ein Gemisch davon, einem öl wie Rizinusöl und einem festen Antioxidationsmittel durch Vermischen der Einzelbestandteile bei einer Temperatur von ≤ 50 °C, bevorzugt 20-50 °C, herstellt und dieses Gemisch bei ≤ 50 °C, bevorzugt 30-50 °C, mit den anderen Bestandteilen, ohne daß Wasser vorhanden ist, in fachmännischer Weise vermischt. Für den Fachmann ist dabei klar, daß zuvor bestimmte Einzelphasen der Formulierung hergestellt werden, die dann in die Mischung mit dem obigen Komplex Polymeres/Öl/Antioxidationsmittel einbezogen werden.

Eine bevorzugte Ausführungsform des Verfahrens besteht darin, daß in dem homogenen Gemisch ein Natriumsalz von Polyacrylsäure oder ein Gemisch von Poly(natriumacrylat)-Homopolymerem und einem Natriumsalz von Polyacrylsäure eingesetzt wird.

Die Erfindung betrifft auch die Verwendung der kosmetischen Formulierung zum Auftragen auf die Haut, insbesondere die Gesichtshaut, oder eines Hautabschnittes einschließlich der Lippen, zur Volumenvergrößerung im Bereich der aufgetragenen Formulierung nach Kontakt mit Feuchtigkeit oder Wasser.

Neben der Volumenvergößerung der erfindungsgemäßen Formulierung sind besondere Vorteile ihre Dauerhaftigkeit, ihr Glättungseffekt, kein "Ausbluten" von Einzelkomponenten tritt auf und ihre ausgezeichnete Hautverträglichkeit. Erstmals in der Kosmetik ist bei Lippenstiften eine Mundbetonung nicht nur durch Farbauftrag möglich, sondern auch dadurch, daß man die Lippen voller erscheinen läßt.

Anwendertests bei Lippenstiften zeigen eine deutliche Akzeptanz. Das Lippengefühl ist nach dem Auftragen angenehm und geschmeidig zugleich mit der Empfindung, daß die Lippen voluminöser erscheinen. Die Konturenfestigkeit ist gegeben, und auch nach längere Tragezeit wird das Lippengefühl noch als überwiegend gut oder sehr gut beurteilt.

Die Erfindung soll nachstehend durch Beispiele näher erläutert werden. Alle Angaben erfolgen in Gewichtsprozent, sofern nichts anderes angegeben ist.

### Beispiel 1 Lippenstift

| **Phase A** | |
|---|---|
| Carnaubawachs | 2 |
| Candelillawachs | 6,5 |
| Ozokerit | 3 |
| Behenylerucat | 2,5 |
| Myristylmyristat | 3 |
| Oleylalkohol | 11 |
| Lanolin | 8 |
| Polydecen | 9,5 |
| Ca-Al-B-Silicat | 2,5 |

| **Phase B** | |
|---|---|
| Rizinusöl | ad 100 |

| **Phase C** | |
|---|---|
| Farben | 8 |
| Rizinusöl+Tocopherylacetat | 11 |

| **Phase D** | |
|---|---|
| Glimmer | 2 |

| **Phase E** | |
|---|---|
| Natriumpolyacrylat | 7 |
| Rizinusöl | 7 |
| Tocopherylacetat | 1 |

| **Phase F** | |
|---|---|
| Parfüm | 1 |

Die Komponenten der Phase A wurden in einem Kessel bei etwa 85 bis 95 °C miteinander vermischt. Unter Beibehaltung der Temperatur auf diesem Niveau wurden die Bestandteile der Phase B hinzugegeben und bis zur gleichmäßigen Verteilung vermischt. Nach Zugabe der Phase C bei 80 bis 85 °C und anhaltendem Mischen wurde die Phase D bei dieser Temperatur zugegeben und der Mischvorgang unter Aufrechterhaltung der Temperatur für 1 Stunde fortgesetzt.

Durch Vermischen der Einzelbestandteile der Phase E wurde eine Dispersion mit einer Viskosität von 7500 bis 8500 Pa·s (cp) hergestellt. Nach ausreichender Dispergierung bei einer Temperatur von ≤50 °C wurde die Phase E dem oben beschriebenen Gemisch der Phasen A bis D bei ebenfalls ≤50 °C hinzugegeben und anschließend bei kurzer Mischzeit von etwa 1 Minute die Phase F hinzugesetzt. Danach wurde das fertige Gemisch in entsprechende Formen gegossen und abgekühlt.

In ähnlicher Weise wurde die folgenden Produkte hergestellt.

### Beispiel 2 Lippenstift

| | |
|---|---|
| Carnaubawachs | 2,5 |
| Candelillawachs | 3,5 |
| Ozokerit | 7 |
| Behenylerucat | 2 |
| Caprylic/Capric Triglyceride | 9 |
| Polydecen | 6,5 |
| Lauryllysin | 2,5 |
| Rizinusöl | ad 100 |
| Farben | 10,5 |
| Glimmer | 5 |
| Natriumpolyacrylat | 5 |
| Tocopherolacetat | 2 |
| Parfüm | 1 |

### Beispiel 3 Cremegrundlage

| | |
|---|---|
| Carnaubawachs | 4,5 |
| Tridecyl Trimellitate | 11,5 |
| Natriumpolyacrylat | 6 |
| Rizinusöl | 6 |
| Butylparaben | 0,06 |
| Cetyaryl Octanoat | ad 100 |
| Magnesiumstearat | 3 |
| Polymethylmethacrylat | 10 |
| Maisstärke | 3 |
| Farben | 4,5 |
| Glimmer | 21 |
| Talkum | 18 |
| SiO₂ (Silica) | 2 |

### Beispiel 4 Wimperntusche

| | |
|---|---|
| Stearinsäure | 9 |
| Montanwachs | 1,5 |
| Bienenwachs | 8,5 |
| Isopropyllanolat | 3,5 |
| Natriumpolyacrylat | 4 |
| PVP/Eicosen Copolymer | 0,5 |
| Rizinusöl | 6,5 |
| Glycerin | 2 |
| Hectorit | 0,3 |
| Acacia Catechu | 2 |
| Farben | 13 |
| Glimmer | 2 |
| Trisamino | 2 |
| Diazolidinylharnstoff | 0,3 |
| Phenoxyethanol | 1 |
| Parfüm | 0,3 |
| Wasser | ad 100 |

### Anwendungsbeispiel

Das folgende Anwendungsbeispiel wurde in Form einer Untersuchung (Studie) des nach Beispiel 1 hergestellten Lippenstiftes Muster 1) sowie eines handelsüblichen Vergleichs-Lippenstiftes Margaret Astor "Raising Sun" (Muster 2) erbracht.

Es wurden modellierte Lippen aus einem zahnärztlichen Gußmaterial verwendet, auf die die Testmuster aufgetragen wurden. Der zur Untersuchung eingesetzte Bildanalysator mit einer speziellen Software arbeitet mit einem Winkel von 90 °, so daß ein Computerbild des Lippenmodells erstellt werden konnte. Dabei wurde von dem Bildanalysator die Dicke des Testmaterials auf den Lippenrändern gemessen. Die dunkle Produktfarbe auf dem Lippenmodell gestattete dem Computer die Produktdicke zu messen durch Bestimmung des Farbkontrastes vom Lippenrand zum Produkt.

Nach Auftragen des Lippenstiftes auf die Modellippen mit einem angefeuchteten Applikator (Baumwolltuch) mit einer Rollbewegung über 15 Sekunden wurde nach einer Wartezeit von 1 Minute der Bildanalysator eingeschaltet. Der feuchte Applikator simulierte zugleich das beim Auftragen eines Lippenstiftes auf natürlichen Lippen übliche Ablecken der Lippen.

Die nach der Aktivierungszeit aufgetretene Dicke der Muster wurde gemessen. Danach wurden die Modellippen abgewaschen, und ein neues Auftragen des Lippenstiftes erfolgte.

Bei 20 Wiederholungsmessungen für einen Lippenstift ergab sich eine mittlere Produktdicke von 196 µm für das Muster 2 und von 289 µm für das Muster 1. Das entspricht einer Volumenvergrößerung von 48 % für das Muster 1. Die statistische Signifikanz bei den Messungen betrug P≤0,05.

## Patentansprüche

1. Rosmetische Formulierung mit Volumeneffekt, **gekennzeichnet durch** einen Gehalt an
a) einem Poly(natriumacrylat)-Homopolymeren, einem Natriumsalz von Polyacrylsäure oder einem Gemisch davon, das bei Kontakt mit Feuchtigkeit oder Wasser sein Volumen bis zm 5-fachen seines ursprünglichen Volumens vergrößert, im Bereich von 0,01 bis 90 Gew-%;
b) einem Erweichungsmittel, umfassend kosmetisch annehmbare öle, Ester, Silikonflüssigkeiten und deren Gemische im Bereich von 0,1 bis 95 Gew-%;
c) Wachs, umfassend Carnaubawachs, Candelillawachs, Ozokerit, Ceresin, Paraffin, synthetischen Wachs und Gemische dieser Wachse im Bereich von 0,1 bis 50 Gew-%;
d) Öl, umfassend Rizinusöl, Lanolinöl, synthetisches öl, Silikonöl und Gemische davon im Bereich von 0,5 bis 90 Gew-%;
e) weitere Wirk- und Hilfsstoffe, wie Antioxidationsmittel, Fette, Fettsäureester, Lanolin, Lanolinderivate, Farben, Pigmente, Parfüm, Schutzmittel, Glimmer, Talkum, Siliciumdioxid, synthetische Harze, Polymere, die einen restlichen Anteil bis zu 100 Gew-% ausmachen;
wobei die Prozentangaben auf die Gesamtformulierung bezogen sind und wobei die Formulierung einen Wassergehalt von kleiner als 0,01 Gew-% hat.

2. Formulierung nach Anspruch 1, **dadurch gekennzeichnet, daß** die kosmetisch annehmbare öle, Ester und Silikonflüssigkeiten Isoeicosan, Dimethicone, Myristylmyristat und Behenylerucat umfassen.

3. Formulierung nach Anspruch 1, **dadurch gekennzeichnet, daß** sie ein separat hergestelltes Gemisch aus Polyacrylat-Polymeren, Rizinusöl und einem festen Antioxidationsmittel enthält.

4. Formulierung nach Anspruch 1, **dadurch gekennzeichnet, daß** sie ein Poly(natriumacrylat)-Polymeres in einer Menge enthält, die zu einer Volumenausdehnung der Gesamtformulierung bei Kontakt mit Feuchtigkeit oder Wasser auf bis zu 150 % des ursprünglichen Volumens führt.

5. Formulierung nach Anspruch 1, **dadurch gekennzeichnet, daß** der Anteil des Poly(natriumacrylat)-Polymeren im Bereich von 0,1 bis 60 Gew-% liegt.

6. Formulierung nach Anspruch 1, **dadurch gekennzeichnet, daß** der Anteil des Poly(natriumacrylat)-Polymeren im Bereich von 0,1 bis 40 Gew-% liegt, vorzugsweise im Bereich von 0,1 bis 20 Gew-%.

7. Formulierung nach Anspruch 1, **dadurch gekennzeichnet, daß** sie in Form eines Lippenstiftes, einer Grundierung, einer Wimperntusche, eines Make-up's, eines Kaschierstiftes vorliegt.

8. Formulierung nach Anspruch 1, **dadurch gekennzeichnet, daß** sie in Form eines Lippenstiftes mit einem Wachsanteil von 8 bis 15 Gew-%, einem ölanteil von 22 bis 32 Gew-%, einem Anteil an Poly(natriumacrylat)-Homopolymerem von 4 bis 12 Gew-%, einem Anteil an Erweichungsmittel von 2 bis 20 Gew-% und dem restlichen Anteil weiterer Wirk- und Hilfsstoffe vorliegt.

9. Formulierung nach Anspruch 1, **dadurch gekennzeichnet, daß** der Anteil des Erweichungsmittels im Bereich von 1 bis 60 Gew-% liegt, vorzugsweise im Bereich von 2 bis 50 Gew-%.

10. Verfahren zur Herstellung einer kosmetischen Formulierung mit Volumeneffekt, **dadurch gekennzeichnet, daß** man ein homogenes Gemisch aus Poly(natriumacrylat)-Homopolymeren einem Natriumsalz von Polyacrylsäure oder einem Gemisch davon Rizinusöl und einem festen Antioxidationsmittel durch Vermischen der Einzelbestandteile bei einer Temperatur von 20 bis 50 °C herstellt und dieses Gemisch bei 30 bis 50 °C mit den anderen Bestandteilen in Abwesenheit von Wasser vermischt.

11. Verfahren nach Anspruch 10, **dadurch gekennzeichnet, daß** in dem Gemisch ein Natriumsalz von Polyacrylsäure oder ein Gemisch von Poly(natriumacrylat)-Homopolymerem und einem Natriumsalz von Polyacrylsäure eingesetzt wird.

12. Verwendung einer kosmetischen Formulierung nach Anspruch 1 zum Auftragen auf die Haut, insbesondere die Gesichtshaut, oder eines Hautabschnittes einschließlich der Lippen, zur Volumenvergrößerung im Bereich der aufgetragenen Formulierung nach Kontakt mit Feuchtigkeit oder Wasser.

## Claims

1. A cosmetic formulation with a swelling effect, which comprises:
a) a poly(sodiumacrylate) homopolymer, a sodium salt of polyacrylic acid or a mixture thereof, which swells up to 5 times beyond its original volume upon contact with moisture or water, the amount of the substance is within a range of 0.01-90 weight percent;
b) an emollient comprising cosmetically-acceptable oils, esters, liquid silicones and their mixtures within a range of 0.1-95 weight percent;
c) a wax comprising carnauba wax, candelilla wax, ozocerite, ceresin, paraffin, synthetic wax and mixtures thereof within a range of 0.1-50 weight percent;
d) an oil comprising castor oil, lanolin oil, synthetic oil, silicone oil and mixtures thereof within a range of 0.5-90 weight percent;
e) additional agents and adjuvants such as antioxidants, fats, fatty acid esters, lanolin, lanolin derivatives, dyes, pigments, perfume, protective agents, mica, talcum, silicon dioxide, synthetic resins, and polymers that comprise a remaining portion of up to 100 weight percent, being all free of water;
whereby the percentages refer to the overall formulation, and whereby the formulation contains less than 0.01 weight percent water.

2. A formulation according to claim 1, wherein the cosmetically acceptable oils, esters, and liquid silicones used comprise isoeicosane, dimethicone, myristyl myristate and behenyl erucate.

3. A formulation according to claim 1, wherein it contains a separately-prepared mixture of polyacrylate/ polymers, castor oil and a solid antioxidant.

4. A formulation according to claim 1, wherein it contains a poly(sodiumacrylate) polymer at an amount that leads to the swelling of the overall formulation up to 150% of the original volume upon contacting moisture or water.

5. A formulation according to claim 1, wherein the amount of poly(sodiumacrylate) polymer ranges from 0.1 to 60 weight percent.

6. A formulation according to claim 1, wherein the amount of poly(sodiumacrylate) polymer ranges from 0.1 to 40 weight percent, preferably in the range of 0.1-20 weight percent.

7. A formulation according to claim 1, wherein it is in the form of a lipstick, foundation, mascara, make-up, or concealer.

8. A formulation according to claim 1, wherein it is in the form of a lipstick with a wax component of 8-15 weight percent, an oil component of 22-32 weight percent, a poly(sodiumacrylate) homopolymer component of 4-12 weight percent, an emollient component of 2-20 weight percent with the remainder being other agents and adjuvants.

9. A formulation according to claim 1, wherein the amount of emollient is 1-60 weight percent, preferably in the range of 2-50 weight percent.

10. A procedure to prepare a cosmetic formulation with a swelling effect wherein a homogenous mixture is prepared consisting of poly(sodiumacrylate) homopolymer or a sodium salt of polyacrylic acid or a mixture thereof, castor oil and a solid anti-oxidant by mixing the individual components at ≤ 50°C, and this mixture is mixed at ≤ 50°C with the other components, all without the presence of water.

11. A procedure according to claim 10, wherein in the mixture is used a sodium salt of polyacrylic acid, or a mixture of poly(sodium acrylate) homopolymer and a sodium salt of polyacrylic acid.

12. Use of a cosmetic formulation according to claim 1 for application on the skin, especially the facial skin or a skin section including the lips to increase the volume of the applied formulation after it contacts moisture or water.

## Revendications

1. Formulation cosmétique à volume variable **caractérisée en ce qu'**elle contient
a) un homopolymère de poly(acrylate sodique), un sel de sodium d'acide polyacrylique ou leur mélange qui, en contact avec de l'humidité ou de l'eau, atteint un volume correspondant à jusqu'à 5 fois son volume initial, en une quantité de 0,01 à 90 % en poids ;
b) un agent assouplissant, incluant des huiles, des esters, des fluides de silicone acceptables du point de vue cosmétique et leurs mélanges en une quantité comprise entre 0,1 et 95 % en poids ;
c) de la cire, incluant cire de carnauba, cire de candellila, ozokérite, cérésine, paraffine, des cires synthétiques et des mélanges de ces cires en une quantité comprise entre 0,1 et 50 % en poids ;
d) une huile, incluant huile de ricin, huile de lanoline, huile synthétique, huile de silicone et leurs mélanges, en une quantité comprise entre 0,5 et 90 % en poids ;
e) d'autres substances actives et adjuvants, comme un agent anti-oxydant, des graisses, des esters d'acide gras, de la lanoline, des dérivés de lanoline, des colorants, des pigments, des parfums, des agents de protection, du mica, du talc, du dioxyde de silicium, des résines synthétiques, des polymères constituant le reste de la formulation jusqu'à 100 %, les pourcentages étant rapportés à la formulation totale et la formulation ayant une teneur en eau inférieure à 0,01 % en poids.

2. Formulation selon la revendication 1 **caractérisée en ce que** les huiles, esters et fluides de silicone acceptables sur le plan cosmétique incluent l'isoéicosane, le diméthicone, le myristate de myristyle et le béhényl érucate.

3. Formulation selon la revendication 1 **caractérisée en ce qu'**elle contient un mélange produit séparément de polymères de polyacrylate, d'huile de ricin et d'un agent anti-oxydant solide.

4. Formulation selon la revendication 1 **caractérisée en ce qu'**elle contient un polymère de poly(acrylate sodique) en une quantité qui entraîne une expansion de volume de la formulation complète lors d'un contact avec de l'humidité ou de l'eau pouvant atteindre 150 % du volume initial.

5. Formulation selon la revendication 1 **caractérisée en ce que** la part du polymère de poly(acrylate sodique) est comprise entre 0,1 et 60 % en poids.

6. Formulation selon la revendication 1 **caractérisée en ce que** la part de polymère de poly(acrylate sodique) est comprise entre 0,1 et 40 % en poids, de préférence entre 0,1 et 20 % en poids.

7. Formulation selon la revendication 1 **caractérisée en ce qu'**elle se présente sous la forme d'un bâtonnet pour les lèvres, d'un fond de teint, d'un mascara, d'un maquillage, d'un bâtonnet camouflant.

8. Formulation selon la revendication 1 **caractérisé en ce qu'**elle se présente sous la forme d'un bâtonnet pour les lèvres contenant une part de cire comprise entre 8 et 15 % en poids, une part d'huile comprise entre 22 et 32 % en poids, un part d'homopolymère de poly(acrylate sodique) comprise entre 4 et 12 % en poids, une part d'agent assouplissant comprise entre 2 et 20 % en poids et le reste étant constitué d'autres substances actives et adjuvants.

9. Formulation selon la revendication 1 **caractérisée en ce que** la part d'agent assouplissant est comprise entre 1 et 60 % en poids, de préférence entre 2 et 50 % en poids.

10. Procédé de fabrication d'une formulation cosmétique à volume variable **caractérisé en ce qu'**on fabrique un mélange homogène d'homopolymère de poly(acrylate sodique), d'un sel sodique d'acide polyacrylique ou de leur mélange, d'huile de ricin et d'un agent anti-oxydant solide en mélangeant les composants individuels à une température de 20 à 50 °C et **en ce qu'**on mélange ce mélange à une température de 30 à 50 °C avec les autres composants en présence d'eau.

11. Procédé selon la revendication 10 **caractérisé en ce que** dans le mélange on utilise un sel sodique d'acide polyacrylique ou un mélange d'homopolymère de poly(acrylate sodique) et d'un sel sodique d'acide polyacrylique.

12. Utilisation d'une formulation cosmétique selon la revendication 1 pour application sur la peau, en particulier sur la peau du visage, ou sur une partie de la peau incluant les lèvres, pour augmenter le volume dans la zone de la formulation appliquée après contact avec de l'humidité ou de l'eau.
